# EUROPEAN PATENT SPECIFICATION

(11) **EP 2 055 274 B1**
(45) Date of publication and mention of the grant of the patent: **27.10.2010**
(21) Application number: 08167777.5
(22) Date of filing: 28.10.2008
(51) Int. Cl.: A61F 2/46

(54) **Taper Sleeve Extractor**
Klebehülsenextraktionsvorrichtung
Extracteur de manchon conique

(30) Priority: 31.10.2007 US 931495
(43) Date of publication of application: 06.05.2009
(73) Proprietor: DePuy Products, Inc., Warsaw, Indiana 46581 (US)
(72) Inventor: Daniels, David W, Winona Lake, IN 46590 (US); Noftz, Rebecca D, Warsaw, IN 46582 (US); Rhoades, Joel C, Pierceton, IN 46562 (US); Huff, Daniel N, Toledo, OH 46562 (US)
(74) Representative: Belcher, Simon James

(56) References cited:
- DE-U1-202006 000 845
- FR-A- 2 606 628
- US-A1- 2004 010 262
- US-A1- 2005 209 597
- US-B1- 6 613 091

## Description

The present invention relates to instruments for use in orthopaedic surgery.

Patients who suffer from the pain and immobility caused by osteoarthritis and rheumatoid arthritis have an option of joint replacement surgery. Joint replacement surgery is quite common and enables many individuals to function properly when it would not be otherwise possible to do so. Artificial joint prostheses usually include components formed from metal, ceramic or plastic materials that are fixed to existing bone.

Such joint replacement surgery is otherwise known as joint arthroplasty. Joint arthroplasty is a well-known surgical procedure by which a diseased and/or damaged joint is replaced with a prosthetic joint. In a typical total joint arthroplasty, the ends or distal portions of the bones adjacent to the joint are resected or a portion of the distal part of the bone is removed and the artificial joint is secured thereto.

There are known to exist many designs and methods for manufacturing implantable articles, such as bone prostheses. Such bone prostheses include components of artificial joints such as elbows, hips, knees and shoulders.

A major critical concern in relation to hip joint replacement is the stability of the implanted joint. Instability is associated with dislocation of the joint.

Factors related to dislocation include surgical technique, implant design, implant positioning and patient related factors. In total hip arthroplasty, implant systems address this concern by offering a series of products with a range of lateral offsets, neck offsets, head offsets and leg lengths. The combination of these four factors affects the laxity of the soft tissue. By optimizing the biomechanics, the surgeon can provide a patient a stable hip that is more resistant to dislocation.

In the case of a damaged hip joint, replacement involves resection of the proximal femur and implantation of the femoral component of an orthopaedic joint, which includes a stem part that can be received in the intramedullary canal, and a head part with a convex bearing surface. The patient's acetabulum is prepared to receive the acetabular component of the joint prosthesis, which provides a concave bearing surface to articulate with the bearing surface on the femoral component. Frequently, bone cement is used to affix the components of the prosthesis within their respective prepared bone cavities.

It can be desirable to retain much of the proximal femur when the condition of the femoral bone tissue is generally good. Techniques have been developed in which the femoral head is fitted within a hollow resurfacing shell. The resurfacing shell has a convex outer surface that is highly polished which enables it to act against the hollow bearing surface of an acetabular component. Such techniques are referred to as Articular Surface Replacement (ASR) techniques. They have the advantage that the quantity of bone that has to be removed from the head of the bone is only small. A tool which can be used to prepare the head in this way is disclosed in WO-2004/032767.

In some ASR kits, there are a large number of femoral heads that can fit on various stem tapers with the use of sleeves. The sleeves have an inner and an outer taper. The inner taper engages with the stem and the outer taper engages with the head. The sleeves also provide various neck offsets to allow for neck length adjustments. Once the surgeon assembles the head and sleeve, it is difficult to disassemble them because of the taper. However, surgeons may sometimes need to do so to achieve better range of motion or a different neck offset. If the surgeon cannot easily remove the sleeve from the head, the surgeon must open another head and sleeve, which leads to waste.

US-A-2005/209597 discloses an instrument for removing a resurfacing femoral component of a hip joint prosthesis which makes of articulating arms to fit over the component and to grip it on the flat face which is directed away from the bearing surface. A pulling force can then be applied to the component to separate it from the head of the femur.

FR-A-2606628 discloses a joint prosthesis component which includes a stem part, a head part, and an intermediate sleeve which is connected to the stem and head parts by means of cooperating tapers.

It is desirable to be able to remove the sleeve from the head of a joint prosthesis component without causing damage to either the head or the sleeve.

The invention provides a kit for use in orthopaedic surgery as defined in claim 1.

Preferred embodiments of the invention are set forth in the dependent claims. The kit can include a plurality of heads. At least two of the plurality of heads have a different diameter. Each of the plurality of heads include a female taper. The kit also includes a plurality of sleeves, at least two of the plurality of sleeves having a different size. Each of the plurality of sleeves having a male taper. The kit also includes an extractor tool. The extractor tool includes a handle and a plurality of tips. Each of the plurality of tips includes a flexible portion.

The instruments of the invention can be used in a method for removing a first orthopaedic component from a second orthopaedic component, in which the first and second orthopaedic components are joined by means of a taper lock. The method includes providing an extractor tool having a handle and an extractor tip that has a flexible portion. The flexible portion is inserted through a bore in the first component. The flexible portion snaps open, and a force is exerted against the handle of the extractor tool, thereby loosening the taper between the first and second components. The first component is held in place while the second component falls away, thereby removing the first component from the second component.

Embodiments of the invention are described below by way of example with reference to the the accompanying drawings, in which:
FIG. 1 is a side view of a orthopaedic implant including a head, a sleeve, and a stem;
FIG. 2 is a cross-sectional view of a head and sleeve engaged in the head;
FIG. 3 is a side view of an extractor tool;
FIG. 4 is a cross-sectional view of the handle;
FIG. 5 is a perspective view of the handle of FIG. 3;
FIG. 6 is an exploded view of the handle of FIG. 3;
FIG. 7 is a perspective view of the extractor tip of FIG. 3;
FIG. 8 is a cross-sectional view of an extractor tip
   inserted into an orthopaedic implant;
FIG. 9 is a flow chart illustrating the use of the extractor tool; and
FIG. 10 is a plan view of a kit.

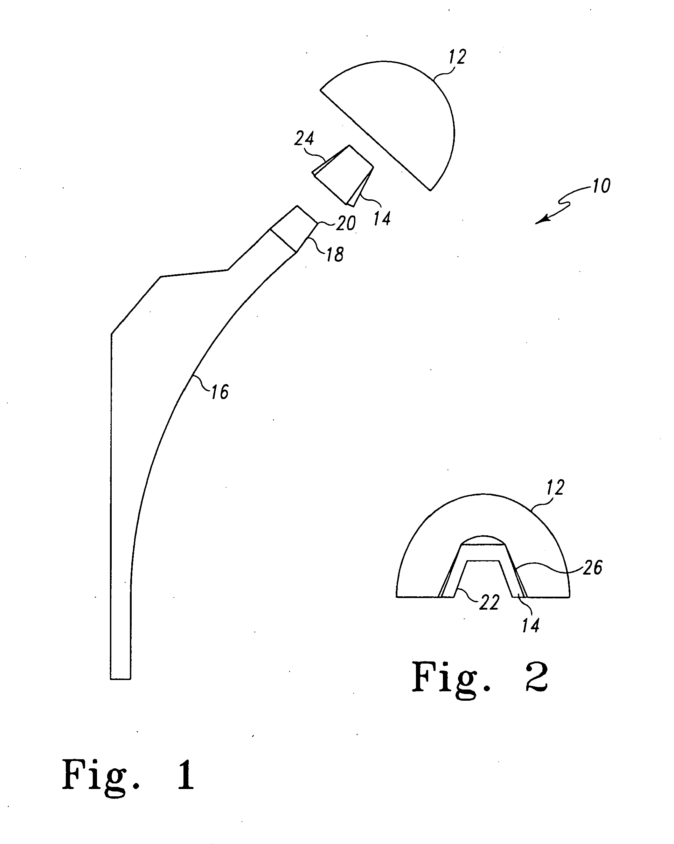

Referring to the drawings, FIG. 1 show an implant 10 which includes a head 12, a sleeve 14, and a stem 16. The stem 16 includes a tapered portion 18. The tapered portion 18 includes a male taper 20 that matches an internal female taper 22 on the sleeve 14 (FIG. 2). In other words, the taper angle of the internal female taper 22 of the sleeve 14 matches the taper angle of the male taper 20 of the stem 16. The sleeve 14 also includes an external male taper 24 that matches a female taper 26 of the head 12 (FIG. 2). In other words, the taper angle of the external male taper 24 of the sleeve 14 matches the taper angle of the female taper 26 of the head 12.

In assembling the head 12, sleeve 14, and stem 16, the surgeon inserts the sleeve 14 into the head 12 and lock the sleeve 14 and the head 12 by means of the tapers 24, 26. However, if the surgeon would need to remove the sleeve 14 from the head 12, the tapers 24, 26 are difficult, if not impossible, to disengage without the use of a tool.

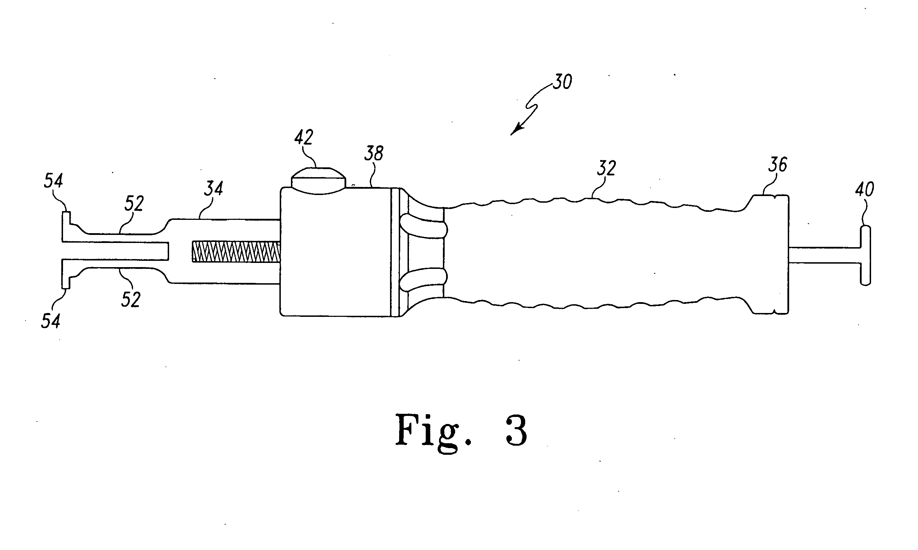

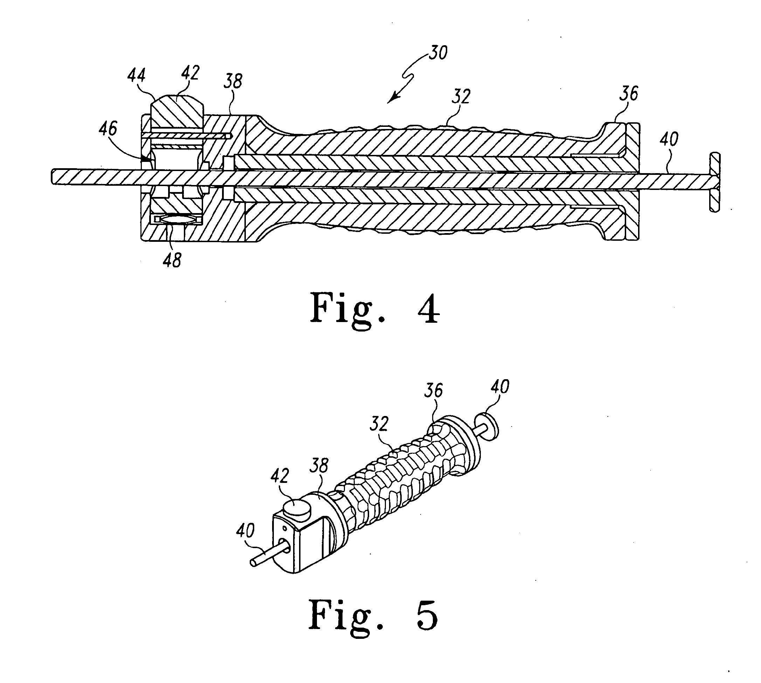

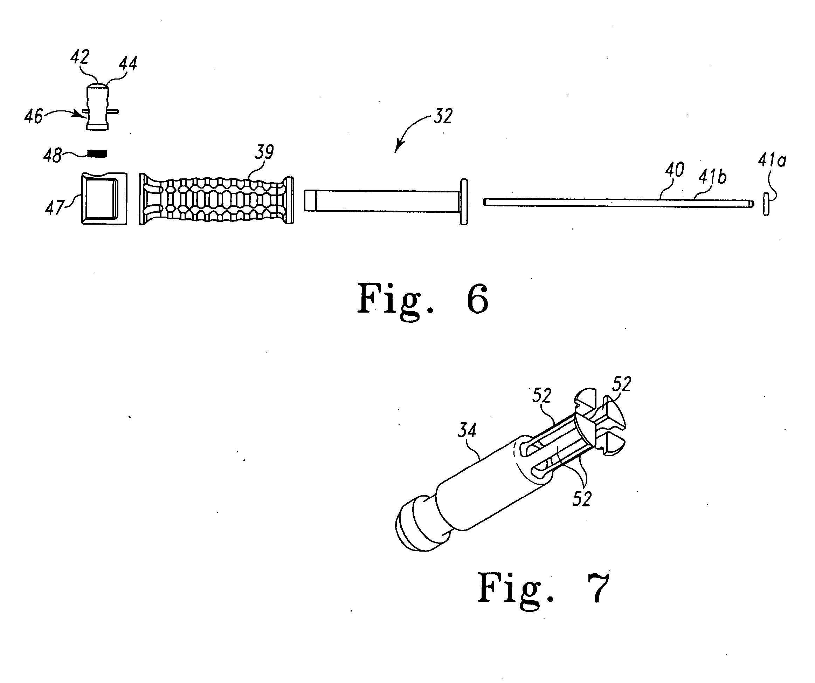

FIG. 3 shows an extractor tool 30 which includes a handle 32 and an extractor tip 34. The handle 32 includes a proximal end 36 and a distal end 38. The handle 32 includes a gripping portion 39. The proximal end 36 includes an impaction mechanism 40. As shown in FIG. 6, the impaction mechanism 40 includes a plate 41a and a shaft 41 b. During use of the tool, the plate 41a would be struck by the surgeon using a tool. As shown in FIGS. 4 and 5, the shaft 41b extends through the length of the handle 32. When the handle 32 is connected to the extractor tip 34, the shaft 41 b will extend into the extractor tip 34, as shown in FIG. 3.

The distal end 38 is connected to the extractor tip 34. A locking mechanism 42 on the handle 32 is used to connect the extractor tip 34 to the handle 32. As shown in the cross-section view of the tool 30 in FIG. 4 the locking mechanism 42 is a spring-loaded button including a button 44, an opening 46, a button housing 47, and a spring 48. As shown in FIG. 4, the locking mechanism 42 engages a recess 50 in the extractor tip 34. The locking mechanism 42 thus holds the extractor tip 34 in a predetermined location. If the user should wish to remove the extractor tip 34, the user presses on the button 44 and releases the extractor tip. When the button 44 is not being activated, the spring 48 exerts a force upward, locking the extractor tip 34 in place. The individual pieces of the locking mechanism 42 are shown in greater detail in the exploded view of the handle 32 in FIG. 6. Although the illustrated locking mechanism is a button utilizing a spring, other known locking mechanisms may be used. For example, the extractor tip 34 could be threaded on or could have a post/slot locking mechanism.

As shown in FIGS. 3, 4, the extractor tip 34 includes a proximal end 51 a and a distal end 51b. At the distal end 51b, the extractor tip 34 includes a flexible portion, which in this embodiment includes a plurality of spaced apart legs 52. Each leg 52 extends generally parallel to a longitudinal axis of the extractor tip 34. Distal to the legs 52, each leg 52 includes a foot 54 that extends generally transverse to the longitudinal axis 55.

Turning now to FIG. 7, the extractor tip 34, and specifically, the legs 52 and feet 54, will be shown in greater detail. As shown in this embodiment, there are four legs 52 and four feet 54. Because of the space between each of the legs 52, the legs are somewhat flexible and can move when a force is applied against them. Although the illustrated embodiment shows four legs and four feet, it should be understood that the number of legs and feet could be varied.

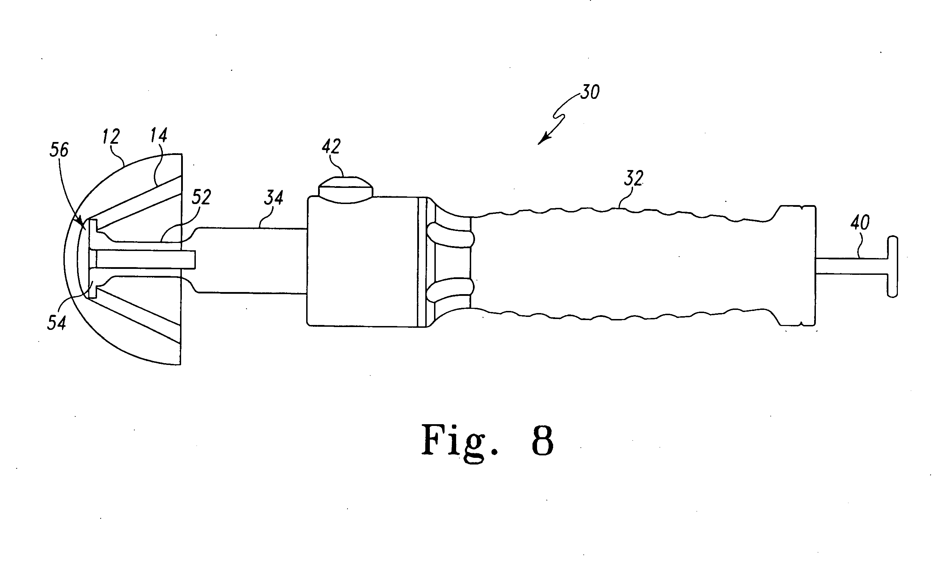

Turning now to FIG. 8, the extractor tool 30 is shown inserted into a head 12 and sleeve 14 that have been locked together. As shown, there is a small gap 56 between the top of the sleeve 14 and the inner portion of the head 12. When the extractor tip 34 is inserted into the sleeve 14, the feet 54 push into the gap 56 and then expand to grasp a top end of the sleeve 14. The impaction mechanism 40 can then be struck by a hammer or other tool, creating a vibration that travels the length of the tool 30 to the feet 54. The vibration at the feet 54 causes the tapers 24, 26 of the head 12 and sleeve 14 to be disengaged, allowing the surgeon to remove the sleeve 14 from the head 12.

The extractor tip 34 may be made of a sterilisable metal such as stainless steel. Other materials may be used, including metals such as aluminium or polymers such as polyphenylsulphone, especially the polyphenylsulphone sold under the trade mark Radel may also be used. The gripping portion 39 of the handle 32 may be made of radel, aluminium, rubber, while the locking mechanism 42, impaction mechanism 40 and strike plate are all made of a sterilisable metal such as stainless steel. In some embodiments the extractor tool 30 may be disposable and all of the parts, except for the shaft 4 1 b may be made of a disposable plastic such as a polyethylene or a polyphenylsulphone.

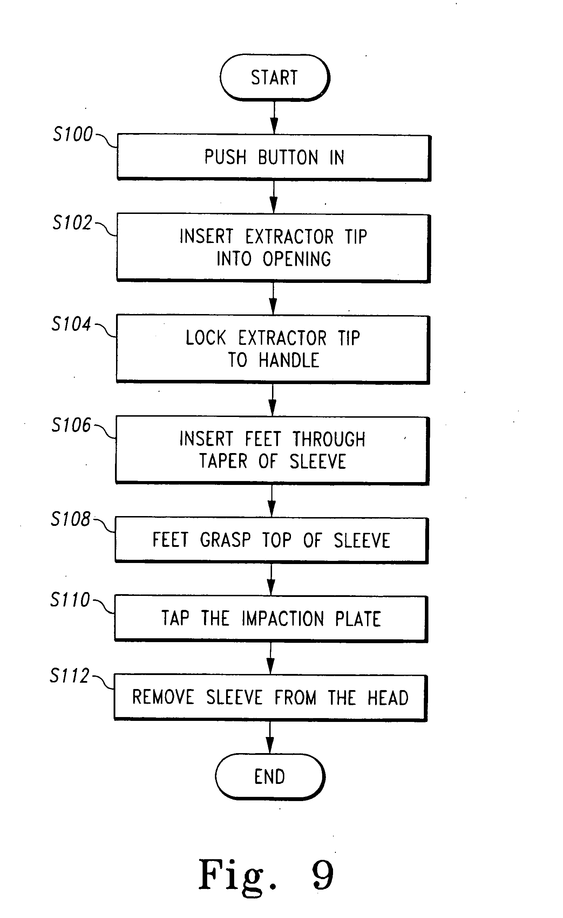

The use of the extractor tool 30 is described below with reference to FIG.9. The surgeon assembles the extractor tool 30 by pushing down the button 44 (s100) while inserting the extractor tip 34 into the opening 46 at step s102. Once the extractor tip 34 is in place, the surgeon releases the button 44, locking the extractor tip 34 to the handle 32 at step s104. The surgeon then pushes the extractor tool 30 against the head 12, thereby inserting the feet 54 of the extractor tip 34 through the internal female taper 22 of the sleeve 14 and into the gap 56 (s 106). Once the feet 54 reach the gap 56 they are no longer under compression, so the feet 54 snap open into the gap 56. Once the impact force is applied, the feet 54 serve as a holding device to keep the sleeve 14 attached to the extractor tip 34 when the head falls away (step s108). Next, at step s110, the surgeon taps the impaction plate 41a with a hammer or other tool (or by hand), creating a force that travels through the handle 32 and into the extractor tool 34. The force sends a vibration through the tapers, thereby disengaging the taper between the external male taper 24 of the sleeve 14 from the female taper 26 of the head 12. The head will fall off the extractor tip, leaving the sleeve still attached at step s112.

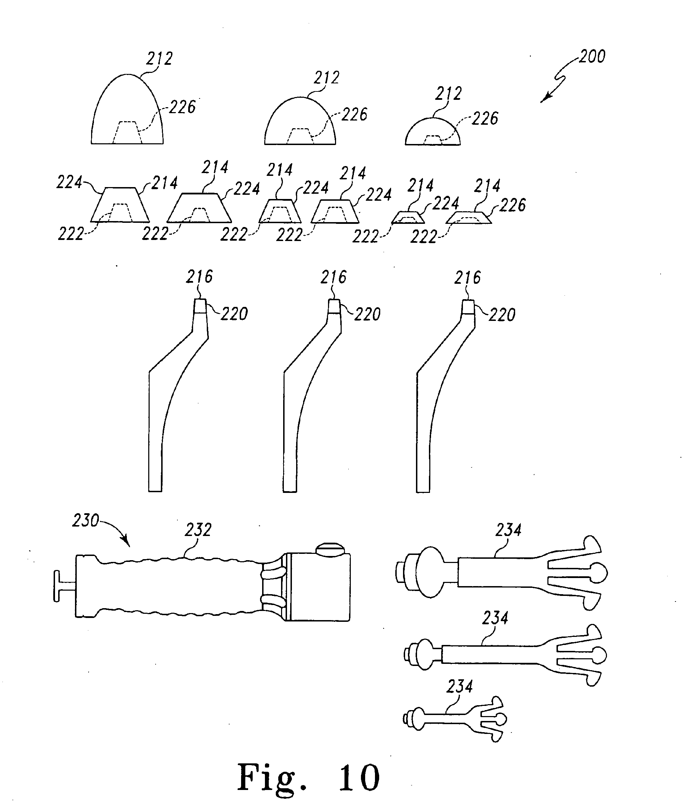

FIG. 10 shows a kit 200 which includes a plurality of heads 212, a plurality of sleeves 214, a plurality of stems 216, and an extractor tool 230. The extractor tool 230 includes a handle 232 and a plurality of extractor tips 234. The diameters of the plurality of heads 212 are different. The heads 212 also include a female taper 226 and the taper angle of these female tapers 226 may also vary. The plurality of sleeves 214 each include an internal female taper 222 and an external male taper 224. The sleeves 214 may vary in size (e.g., length, width, etc...) as well as in taper angle. The tapers of the stems 216 may also vary.

The extractor tips 234 each include a flexible end having a plurality of legs 252, each of the legs 252 having a foot 254. The extractor tips 234 may also vary in length and in width. Because the length of the sleeves 214 may vary, the length of the legs 254 on the various extractor tips 234 may vary to fit in the selected sleeve. Also, because the taper angles of the female taper 226 of the head 212 may vary, the size of the feet 254 and/or the diameter of the extractor tip 234 may also vary. The extractor tips 234 may also vary the number of both the legs 252 and the feet 254.

In use, the surgeon would select one of the plurality of heads 212 and one of the plurality of sleeves 214 and assemble them for use. Should the surgeon need to disassemble the sleeve 214 and the head 212, the surgeon would then select an extractor tip 234 from the plurality of tips 234. The selected extractor tip would have the appropriate length and width to fit into the sleeve 214. The surgeon would then lock the selected extractor tip 234 into the handle and proceed as described above with reference to FIG. 9.

## Claims

1. A kit for use in orthopaedic surgery, which comprises:
(a) an implant assembly (10) comprising a first component (14) and a second component (12) having a recess (26) in which the first component can be received and be locked to the second component by means of a taper lock (24, 26) , in which the first component has a bore extending through it, and
(b) an extractor (30) for extracting the first component of the implant assembly from within the second component, the extractor comprising:
a handle (32),
an extractor tip (34) having a proximal end (51a) and a distal end (51b), the proximal end adapted to connect to the handle, the distal end provided by a plurality of flexible legs (52), each having an outwardly extending foot (54) at its distal end, in which the feet can fit through the bore in the first component when the legs are deformed inwardly, and the feet spring outwardly into a space within the second component between the first component and the second component when they have passed through the bore, and
an impactor mechanism (40) by which an impaction force can be applied to create a vibration which travels along the extractor to the feet so that the vibration causes the taper lock to be released allowing the first and second components to be separated.

2. A kit as claimed in claim 1, in which the plurality of legs (52) are equidistant from one another.

3. A kit as claimed in claim 1, in which the extractor includes four legs (52).

4. A kit as claimed in claim 1, in which the second component is a head (12) having a convex bearing surface, and the first component is a sleeve (14).

5. A kit as claimed in claim 4, which includes:
a plurality of heads (12), at least two of the plurality of heads having different diameters,
a plurality of sleeves (14), at least two of the plurality of sleeves having different sizes, and
a plurality extractor tips (34).

6. A kit as claimed in claim 1, in which the impaction mechanism (40) includes a impact plate (41a) and an impact shaft, the impact shaft extending the length of the handle (32).

7. A kit as claimed in claim 1, in which the handle (32) includes a locking mechanism (42) to lock the tip (34) into the handle.

8. A kit as claimed in claim 7, in which the locking mechanism (42) is a spring-loaded button (44).

9. A kit as claimed in claim 1, in which the extractor tip (34) is made of a stainless steel.

10. A kit as claimed in claim 1, in which the handle (32) includes a gripping portion (39) and the gripping portion is made of a polyphenylsulphone.

## Patentansprüche

1. Ein Bausatz zur Verwendung in der orthopädischen Chirurgie, umfassend:
(a) eine Implantatanordnung (10), die eine erste Komponente (14) und eine zweite Komponente (12) mit einer Aussparung (26) aufweist, in der die erste Komponente aufgenommen werden kann und mit der zweiten Komponente durch eine Taper-Lock-Verbindung (24, 26) arretiert werden, wobei die erste Komponente eine Bohrung aufweist, die sich dort hindurch erstreckt, und
(b) einen Extrahierer (30) zum Extrahieren der ersten Komponente der Implantatanordnung aus der zweiten Komponente, wobei der Extrahierer umfasst:
einen Griff (32),
eine Extrahiererspitze (34), die ein proximales Ende (51 a) und ein distales Ende (51b) aufweist, wobei das proximale Ende zum Verbinden mit dem Griff gestaltet ist,
wobei das distale Ende mit einer Vielzahl von flexiblen Beinen (52) versehen ist, von denen jedes einen sich nach außen erstreckenden Fuß (54) an seinem distalen Ende aufweist, wobei die Füße durch die Bohrung in der ersten Komponente passen können, wenn die Beine nach innen verformt werden, und die Füße nach außen in einen Raum in der zweiten Komponente zwischen der ersten Komponente und der zweiten Komponente springen, wenn sie durch die Bohrung gegangen sind, und einen Stoßmechanismus (40), durch den eine Stoßkraft ausgeübt werden kann, um eine Vibration zu erzeugen, die entlang dem Extrahierer zu den Füßen verläuft, so dass die Vibration die Taper-Lock-Verbindung dazu veranlasst, gelöst zu werden, um zu ermöglichen, dass die ersten und zweiten Komponente getrennt werden.

2. Bausatz nach Anspruch 1, bei dem die Vielzahl von Beinen (52) voneinander äquidistant sind.

3. Bausatz nach Anspruch 1, bei dem der Extrahierer vier Beine (52) enthält.

4. Bausatz nach Anspruch 1, bei dem die zweite Komponente ein Kopf (12) ist, der eine konvexe Lagerfläche aufweist, und die erste Komponente eine Hülse (14) ist.

5. Bausatz nach Anspruch 4, der enthält:
eine Vielzahl von Köpfen (12), wobei wenigstens zwei der Vielzahl von Köpfen unterschiedliche Durchmesser aufweisen,
eine Vielzahl von Hülsen (14), wobei wenigstens zwei der Vielzahl von Hülsen unterschiedliche Größen aufweisen, und
eine Vielzahl von Extrahiererspitzen (34).

6. Bausatz nach Anspruch 1, bei dem der Stoßmechanismus (40) eine Stoßplatte (41a) und einen Stoßschaft aufweist, wobei sich der Stoßschaft über die Länge des Griffs (32) erstreckt.

7. Bausatz nach Anspruch 1, bei dem der Griff (32) einen Arretiermechanismus (42) enthält, um die Spitze (34) in den Griff zu arretieren.

8. Bausatz nach Anspruch 7, bei dem der Arretiermechanismus (42) eine federbelastete Taste (44) ist.

9. Bausatz nach Anspruch 1, bei dem die Extrahiererspitze (34) aus Edelstahl hergestellt ist.

10. Bausatz nach Anspruch 1, bei dem der Griff (32) einen Greifabschnitt (39) enthält, und der Greifabschnitt aus Polyvenylsolphon besteht.

## Revendications

1. Kit destiné à être utilisé en chirurgie orthopédique, qui comprend :
(a) un ensemble d'implant (10) comprenant un premier composant (14) et un deuxième composant (12) comportant un évidement (26) dans lequel le premier composant peut être reçu et être verrouillé au deuxième composant au moyen d'un verrou conique (24, 26), dans lequel le premier composant comporte un alésage s'étendant à travers celui-ci, et
(b) un extracteur (30) pour extraire le premier composant de l'ensemble d'implant de l'intérieur du deuxième composant, l'extracteur comprenant :
■ une poignée (32),
■ un embout d'extracteur (34) comportant une extrémité proximale (51a) et une extrémité distale (51b), l'extrémité proximale étant adaptée pour être reliée à la poignée, l'extrémité distale étant réalisée par une pluralité de jambes (52) flexibles, comportant chacune un pied (54) s'étendant vers l'extérieur à son extrémité distale, dans lequel les pieds peuvent s'insérer à travers l'alésage dans le premier composant lorsque les jambes sont déformées vers l'intérieur, et les pieds sortent élastiquement vers l'extérieur dans un espace dans le deuxième composant entre le premier composant et le deuxième composant lorsqu'ils sont passés à travers l'alésage, et
■ un mécanisme de frappe (40) par lequel une force de frappe peut être appliquée pour créer une vibration qui se propage le long de l'extracteur jusqu'aux pieds de sorte que la vibration provoque la libération du verrou conique, ce qui permet de séparer les premier et deuxième composants.

2. Kit selon la revendication 1, dans lequel la pluralité de jambes (52) sont à égales distances les unes des autres.

3. Kit selon la revendication 1, dans lequel l'extracteur comprend quatre jambes (52).

4. Kit selon la revendication 1, dans lequel le deuxième composant est une tête (12) comportant une surface de support convexe, et le premier composant est un manchon (14).

5. Kit selon la revendication 4, qui comprend :
■ une pluralité de têtes (12), au moins deux de la pluralité de têtes ayant des diamètres différents,
■ une pluralité de manchons (14), au moins deux de la pluralité de manchons ayant des tailles différentes, et
■ une pluralité d'embouts d'extracteur (34).

6. Kit selon la revendication 1, dans lequel le mécanisme de frappe (40) comprend une plaque de frappe (41a) et un arbre de frappe, l'arbre de frappe s'étendant sur la longueur de la poignée (32).

7. Kit selon la revendication 1, dans lequel la poignée (32) comprend un mécanisme de verrouillage (42) pour verrouiller l'embout (34) dans la poignée.

8. Kit selon la revendication 7, dans lequel le mécanisme de verrouillage (42) est un bouton sollicité par ressort (44).

9. Kit selon la revendication 1, dans lequel l'embout d'extracteur (34) est réalisé en acier inoxydable.

10. Kit selon la revendication 1, dans lequel la poignée (32) comprend une partie de préhension (39) et la partie de préhension est réalisée en polyphényle sulfone.
